(19) **Europäisches Patentamt / European Patent Office / Office européen des brevets**

(11) **EP 4 226 883 A1**

(12) **EUROPEAN PATENT APPLICATION**

(43) Date of publication:
**16.08.2023 Bulletin 2023/33**

(21) Application number: **22156800.9**

(22) Date of filing: **15.02.2022**

(51) International Patent Classification (IPC):
**A61B 18/20** (2006.01)

(52) Cooperative Patent Classification (CPC):
**A61B 18/203;** A61B 2018/00476

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **GUJRAL, Rashi Kumra**
  **Eindhoven (NL)**
- **PATIL, Ravindra**
  **Eindhoven (NL)**
- **GUTTA, Srinivas**
  **Eindhoven (NL)**

(74) Representative: **Philips Intellectual Property & Standards**
**High Tech Campus 52**
**5656 AG Eindhoven (NL)**

(54) **APPARATUSES AND METHODS FOR USE WITH A TREATMENT DEVICE**

(57) The present invention relates to apparatuses and methods for use with a treatment device configured to perform a treatment operation on a body part of a subject, wherein the treatment device is configured to apply light pulses to skin of the body part to perform the treatment operation and to output a flash sound per light pulse. A trained algorithm or computing system is used in an embodiment to reliably detect the flash sounds and inform the user to avoid overtreatment.

FIG.1

EP 4 226 883 A1

**Description**

FIELD OF THE INVENTION

The present invention relates to apparatuses and methods for use with a treatment device that is configured to perform a treatment operation on a body part of a subject, wherein the treatment device is configured to apply light pulses to skin of the body part to perform the treatment operation and to output a flash sound per light pulse. The present invention relates further to computer-implemented methods of such apparatuses, a system comprising a treatment device and a computer program.

BACKGROUND OF THE INVENTION

Techniques for removal of unwanted hairs include shaving, electrolysis, plucking, laser and light therapies (known as photoepilation) and injection of therapeutic anti-androgens. Light-based technologies are also used in other types of dermatological treatments, including hair growth reduction and treating acne.

Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. Home-use photoepilation devices, for example the Philips Lumea device, use intense pulsed light (IPL) from high intensity light sources, e.g. Xenon flash lamps that produce high output bursts of broad spectrum light.

A photoepilation treatment is characterized by the user of the photoepilation device treating relatively small areas of the skin for the purpose of hair removal. The photoepilation treatment uses intense light to heat melanin in hair and hair roots, which puts the hair follicles into a resting phase, preventing hair re-growth. For effective use of this technology for hair removal, the user must treat the skin completely without leaving any gaps. Since this effect is only of limited duration, treatment has to be repeated on a regular basis: typically, once every 4 to 8 weeks in the maintenance phase after an initial period of about two months in which treatment is performed once every two weeks.

The Lumea device is supported by a Lumea app (i.e., an application program or software application than can be run on a computer, laptop, tablet and/or smartphone, which can be coupled, e.g. wirelessly, to the treatment device) to aid the user with hair removal treatment. This enhances the user experience and user engagement with the Lumea device.

In many photoepilation devices, each flash/burst of light emitted for treatment is immediately followed by a flash sound, which is a short sound impulse specific in both time (e.g. of approximately one second) and frequency (e.g. of approximately 9 kHz). The number of flashes required for treatment depends on the size of the body part where the pulses are to be applied. The exposure to high intensity laser pulses for a duration beyond the recommended amount can cause to overtreatment and serious damages to the skin.

SUMMARY OF THE INVENTION

It is an object of the present invention to provide measures to avoid or at least reduce the risk of overtreatment.
In a first aspect of the present invention an apparatus is presented for use with a treatment device configured to perform a treatment operation on a body part of a subject, wherein the treatment device is configured to apply light pulses to skin of the body part to perform the treatment operation and to output a flash sound per light pulse, the apparatus comprising

- an input configured to obtain an audio signal sensed during a treatment operation;
- a processing unit configured to

  - segment the audio signal into audio segments, in particular into audio segments of a set or predetermined duration,
  - extract, per audio segment, one or more characteristic features from the audio segments,
  - detect, per audio segment, if the audio segment comprises a flash sound by applying a trained algorithm or computing system that has been trained on a plurality of audio segments each including one or more of the characteristic features to detect flash sounds in the audio segments, and
  - change a flash sound counter counting the number of detected flash sounds since start of a treatment operation or a set or user-instructed moment; and

- an output configured to output one or more of:

- the flash sound counter,
- an indication if the flash sound counter exceeds a flash sound threshold, and
- an indication that the treatment operation has finished.

In a second aspect of the present invention an apparatus is presented for use with a treatment device, the apparatus comprising

- an input configured to obtain an audio signal sensed during a treatment operation or artificially created;
- a processing unit configured to

    - segment the audio signal into audio segments, in particular into audio segments of a set or predetermined duration,
    - extract, per audio segment, one or more characteristic features from the audio segments,
    - derive, per audio segment, from an annotation of the audio signal flash sound information indicating if an audio segment comprises a flash sound, the annotation of the audio signal indicating flash sounds within the audio signal, in particular temporal information of flash sounds, and
    - provide, per audio segment, the extracted one or more characteristic features and the derived flash sound information to an algorithm or computing system to train it for the detection of a flash sound within an audio segment based on one or more characteristic features.

In a third aspect of the present invention an apparatus is presented for use with a treatment device, the apparatus comprising an output configured to control a user interface to:

- display a first screen requesting user input regarding a treatment operation;
- obtain user input via the first screen, the user input comprising an image of a body part of a subject to be treated and/or size information of the size of the body part of the subject to be treated;
- display a second screen indicating connectivity of the apparatus or an electronic device comprising the apparatus with the treatment device;
- display a third screen indicating at least one of:

    - an operation status of the skin treatment device,
    - a flash sound counter counting the number of detected flash sounds since start of a treatment operation or a set or user-instructed moment,
    - an indication if the flash sound counter exceeds a flash sound threshold, and
    - an indication that the treatment operation has finished.

In a fourth aspect of the present invention a system is presented comprising:

a treatment device comprising one or more light sources configured to generate light pulses for application to skin of a subject's body to perform a treatment operation, wherein the treatment device is configured to generate a flash sound per light pulse;
an audio sensor configured to sense an audio signal during a treatment operation; and
an apparatus according to any one of the different aspects as disclosed herein.

In yet further aspects of the present invention, there are provided a corresponding (computer-implemented) methods, a computer program which comprises program code means for causing a computer to perform the steps of the respective methods disclosed herein when said computer program is carried out on a computer as well as a non-transitory computer-readable recording medium that stores therein a computer program product, which, when executed by a processor, causes the respective methods disclosed herein to be performed.

Preferred embodiments of the invention are defined in the dependent claims. It shall be understood that the claimed methods, system, computer program and medium have similar and/or identical preferred embodiments as the claimed apparatuses, in particular as defined in the dependent claims and as disclosed herein.

All aspects of the present invention are based on the common idea that, considering the detrimental aspects of laser hair removal treatment, the user should precisely know the number of flashes required for the treatment and/or should know when the treatment of a desired area is finished and has been treated with a sufficient number of flashes. A single treatment typically takes e.g. around 30-40 flashes (depending on the length of the desired treatment area). Users may manually count the number of flashes applied during one treatment session, and there is no recommendation of any number of flashes which leads to poor user experience. However, users may be otherwise occupied with other tasks or disturbed by background noise so that they may have difficulty to accurately keep track of the total number of flashes. Automating the counting of flash sounds (and, thus, the number of emitted flashes) during a treatment session is thus provided by the present invention (first aspect) using a trained algorithm or computing system. Such a trained algorithm or computing system can be trained to precisely count the total number of flashes during each treatment and deal with background noise originating from both the treatment device and the environment. Further, the aperiodic nature of the signals may be taken into account. Thus, the number of flashes emitted by the skin treatment device during a treatment session can be obtained with improved accuracy.

In the context of the present invention changing a counter for counting the number of flash sounds during a treatment shall be understood such that, each time a flash sound is detected, the counter value is incremented (e.g. starting from zero) or decremented (e.g. starting from an estimated maximum counter value for treating a certain treatment area).

The training is made according to the present invention (second aspect) by use of a plurality of audio segments each including one or more characteristic features derived from an audio segment (e.g. a segment of a duration of e.g. one second) to detect flash sounds in the audio segments.

Further, according to the present invention (third aspect) a user interface is provided that efficiently obtains and presents various information to the user, including the number of flashes emitted during the treatment session. It helps the user to easily understand the current progress of the treatment and to avoid overtreatment.

Preferably, the present invention uses a learning system, machine learning, a classification model, a binary classification model, a neural network, or a convolutional neural network as trained algorithm or computing system. For instance, in an implementation an XGBoost classifier, as conventionally known, may be used. XGBoost is a decision-tree-based ensemble Machine Learning algorithm that uses a gradient boosting framework. It can be used for a wide range of applications, e.g. to solve regression, classification, ranking, and user-defined prediction problems. It may provide a high prediction performance at minimum processing time, both in the training phase (when the system/algorithm is trained) as well as in the operation phase (when the system/algorithm is used for flash sound detection).

In another embodiment one or more features related to time, amplitude and frequency of the audio segment may be used as characteristic feature(s), which may be extracted from the respective audio segment.

Generally, a wide range of features is available for use as characteristic feature(s) of an audio segment, including one or more of start time, end time, amplitude, gradient, instrumentation, key chords, melody, rhythm, pitch, energy, note offset, one or more mel-frequency cepstral coefficients (MFCCs), mel spectrogram, spectral centroid, spectral flux, zero crossing rate, beat, timbre, pitch, harmony, root-mean-square energy, amplitude envelope, band energy ratio, spectrogram, constant Q transform, spectral roll off, and spectral spread. In an implementation selected MFCC features with 8-13 cepstral coefficients may be used instead of a much larger number of e.g. 39 MFCC features that are generally available for an audio segment. Preferably, a zeroth coefficient that represents the average log-energy of the input signal carrying only little speaker-specific information is not used as it may lead to the detection of background noise (the flash sound is generally transient ~0.1 sec). Thus, according to the present invention flash sounds with low amplitude and short duration that are produced by the photoepilation device in the presence of varied background noise can be effectively detected. Further, the required low latency and memory can be provided.

A flash sound may be generated by triggering the light source such as a flash lamp or it may be synthesized artificially in case a light source such as LED is used. For instance, a flash lamp or a xenon arc lamp during operation creates sounds (representing the flash sounds) due to the pulsed discharge. Laser radiation can cause electric breakdown in the air, which produces sound. Similarly, when intense pulsed light comes in contact with skin sound is generated. However, other embodiments of treatment devices may use semiconductor light sources such as LEDs that do not produce a discharge sound since they function differently. An additional sound generator can then be used to synthesize the flash sound each time a light pulse for treatment is emitted.

According to another embodiment of the first aspect the processing unit may be configured to arrange the extracted features related to time, amplitude and frequency of the audio segment into an array and to subject the array to the trained algorithm or computing system to detect, per audio segment, if the audio segment comprises a flash sound. The array format may e.g. be configured such that the extracted features are arranged or sorted according to feature that are related to time, related to amplitude and related to frequency, which makes it more easy or even possible to process them with a trained algorithm or computing system, such as an XGBoost classification model.

Applying the trained algorithm or computing system onto an audio segment by the processing unit may include building classification boundaries based on the one or more characteristic features. XGBoost, as an example, uses an ensemble tree-based technique to build classification boundaries based on the input feature passed. It uses parallel processing, tree-pruning and regularization to avoid overfitting and to provide a balanced model. Based on the classification boundaries each audio segment can be classified as either flashes and non flashes so that finally the total number of flashes can be predicted during a treatment.

In another embodiment of the first aspect the processing unit is configured to use the result of the detection for further training of the algorithm or computing system. Hence, during operation the detection results are not only used for flash sound detection, but at the same time for further training to further improve the trained algorithm or computing system.

According to the second aspect of the present invention, an audio signal sensed during a treatment operation or artificially created is used for training the algorithm or computing system. In the same manner as done during the actual treatment operation, the audio signal is segmented into audio segments and one or more characteristic features are extracted from the audio segments. From an annotation of the audio signal flash sound information indicating if an audio segment comprises a flash sound is derived from the one or more characteristic features. In this context, the annotation of the audio signal shall be understood broadly as indication of flash sounds within the audio signal, in particular temporal information of flash sounds (e.g. a time stamp or other indication that indicates at which point in time the audio signal comprises a flash sound, such as start time, end time, peak time, time duration, etc.). The extracted characteristic features and the derived flash sound information are then used to train the algorithm or computing system for the detection of a flash sound within an audio segment based on one or more characteristic features.

The annotation of the audio signal may be previously made by a user so that the input can obtain the annotation of the audio signal, as e.g. stored along with or separate from the audio signal. Alternatively or in addition, an annotation can be made automatically, e.g. by the algorithm or computing system or another processing entity. For instance, the processing unit may be configured to generate the annotation of the audio signal, e.g. automatically or based on user input.

According to the third aspect of the present invention different invention may be obtained and presented to the user. This information may be displayed on various screens, where a first screen requests user input regarding a treatment operation, a second screen indicates connectivity of the apparatus or an electronic device comprising the apparatus with the treatment device, and a third screen indicates one or more of an operation status of the skin treatment device, a flash sound counter counting the number of detected flash sounds since start of a treatment operation or a set or user-instructed moment, an indication if the flash sound counter exceeds a flash sound threshold, and an indication that the treatment operation has finished. This enables a user to know the current progress of a treatment operation and when to finish the treatment so that overtreatments can be avoided or at least reduced.

The system according to the present invention comprises a treatment device, an audio sensor and one or more of the apparatuses as described above. According to an embodiment of the system the audio sensor, e.g. a microphone, is part of the apparatus or an electronic device comprising the apparatus, in particular part of a computer, laptop, tablet or smartphone (or a separate entity representing only the apparatus). In another embodiment the apparatus is part of the treatment device or an electronic device, in particular a computer, laptop, tablet or smartphone. Generally, the apparatuses of the above-described aspects may be implemented on the same device or on different devices. In a practical implementation, the apparatuses and methods of all aspects are implemented on the same device, such as a tablet or smartphone.

BRIEF DESCRIPTION OF THE DRAWINGS

These and other aspects of the invention will be apparent from and elucidated with reference to the embodiment(s) described hereinafter. In the following drawings

Fig. 1 shows an illustration of an exemplary embodiment of a treatment device with which the invention can be used;
Fig. 2 shows a schematic diagram of an exemplary embodiment of a system according to the present invention;
Fig. 3 shows a flowchart of an embodiment of a method according to a first aspect of the present invention;
Fig. 4 shows a flowchart of an embodiment of a method according to a second aspect of the present invention;
Fig. 5 shows a visualization of an audio signal with indications of flash sounds and background noise;
Fig. 6 shows zoomed-in visualization on two flash sounds;
Fig. 7 shows a schematic diagram illustrating pre-processing according to the present invention;
Fig. 8 shows a schematic diagram illustrating feature extraction according to the present invention;
Fig. 9 shows a flowchart of the process from model deployment to production according to the present invention;
Fig. 10 shows a flowchart of an embodiment of a method according to a third aspect of the present invention; and
Fig. 11 shows different screens illustrating a user interface according to the present invention.

DETAILED DESCRIPTION OF THE EMBODIMENTS

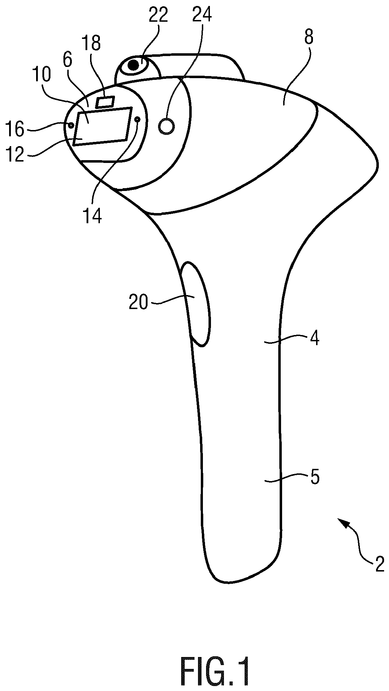

Fig. 1 is an illustration of an exemplary treatment device 2 that can be used to apply a light pulse (also called flash) to an area of skin. It will be appreciated that the treatment device 2 in Fig. 1 is merely presented as an example of a hand-held treatment device 2 that the invention can be used with, and the treatment device 2 is not limited to the form shown in Fig. 1 or to being a hand-held treatment device. The treatment device 2 is for use on a body of a subject (e.g. a person or an animal), and is to be held in one or both hands of a user during use. The treatment device 2 is to perform some treatment operation to the skin or body of the subject using one or more light pulses when the treatment device 2 is in contact with, or close to, a body part of the subject. The treatment operation can be removal of unwanted hairs by laser and/or light therapies (known as a photoepilation treatment or Intense Pulsed Light, IPL, treatment). Alternatively, the treatment operation can be laser and/or light therapies for reasons other than removal or preventing growth of hair, such as a dermatological (skin) treatment, treating acne, a phototherapy treatment for other types of skin condition, skin rejuvenation, skin tightening, or port-wine stain treatment.

As described herein, the treatment device 2 is operated or used by a 'user', and the treatment device 2 is used on a body of a 'subject'. In some cases, the user and the subject is the same person, i.e. the treatment device 2 is held in a hand and used by a user on themselves (e.g. used on the skin on their leg). In other cases, the user and the subject are different people, e.g. the treatment device 2 is held in a hand and used by a user on someone else. In either case, it is difficult for a user to achieve complete coverage of a body part and/or avoid over-treating certain areas of the body part since there are little or no user-perceptible changes to the skin on applying or shortly after applying a light pulse.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed in to contact with the subject in order for the treatment operation to be performed on the body or skin of the subject at the position that the head portion 6 is in contact with the body or skin.

The treatment device 2 is for performing a treatment operation using light pulses. Thus, in Fig. 1 the head portion 6 comprises an aperture 10 arranged in or on the housing 4 so that the aperture 10 can be placed adjacent to or on (i.e. in contact with) the skin of the subject. The treatment device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10. The aperture 10 may be in the form of an opening at the head end 8 of the housing 4, or it may be in the form of a window (including a waveguide) that is transparent or semitransparent to the light pulses (i.e. the light pulses can pass through the window).

In the exemplary embodiment shown in Fig. 1, the aperture 10 has a generally rectangular shape, which results in a generally rectangular-shaped skin treatment region on the skin. It will be appreciated that the aperture 10 can have any other desired shape. For example, the aperture 10 can be square, elliptical, circular, or any other polygonal shape.

Although not shown in Fig. 1, the treatment device 2 may have one or more removable attachments for the head portion 6 of the treatment device 2 that each includes an aperture 10. The attachments may be for use on different body parts, and have apertures 10 with different sizes. For example, one attachment can be for use on a large body part such as the legs and therefore have a large aperture 10, whereas another attachment can be for use on the skin/hair above the upper lip and therefore have a small aperture 10.

The one or more light sources 12 can generate light pulses of any suitable or desired wavelength (or range of wavelengths) and/or intensities. For example, the light source 12 can generate visible light, infra-red (IR) light and/or ultraviolet (UV) light. Each light source 12 can comprise any suitable type of light source, such as one or more light emitting diodes (LEDs), a (Xenon) flash lamp, a laser or lasers, etc. The light source(s) 12 can provide light pulses with spectral content in the 450 -1200 nm range for a duration of around 2.5 ms, as these wavelengths heat melanin in the hair and hair root by absorption, which puts the hair follicles in a resting phase, preventing hair regrowth.

The one or more light sources 12 are configured to provide pulses of light. That is, the light source(s) 12 are configured to generate light at a high intensity for a short duration (e.g. less than 1 second). The intensity of the light pulse should be high enough to effect the treatment operation on the skin or body part adjacent the aperture 10.

The illustrated treatment device 2 may optionally include two skin contact sensors 14, 16 positioned on or in the head portion 6 that are used to determine whether the head portion 6 is in contact with the skin. The skin contact sensors 14, 16 measure a parameter that is indicative of whether the head portion 6 is in contact with skin, and generate respective measurement signals that comprise a time-series of measurements of the parameter. The measurement signals can be processed to determine if the head portion 6 is in contact with skin. Typically, a skin contact sensor is used in a treatment device 2, particularly a photoepilator, to make sure that the treatment device 2 is correctly in contact with skin before a light pulse is generated to avoid the light pulse being directed into the eyes of the user or subject.

In some embodiments the parameter can be capacitance, and so the skin contact sensors 14, 16 can measure capacitance via a respective pair of electrical contacts or electrodes on the surface of the head portion 6, with the measured capacitance being indicative of whether there is skin contact. In alternative embodiments, the parameter can be an intensity or level of light, and so the skin contact sensors 14, 16 can be light sensors that measure an intensity or level of light incident on the light sensor, with the measured intensity or level being indicative of whether there is skin contact (e.g. less/no light could indicate skin contact as the skin obscures the light sensors 14, 16, and vice versa). In other alternative embodiments, the parameter can be a measure of contact pressure, and so the skin contact sensors 14, 16 can measure contact pressure via respective pressure sensors or mechanical switches, with the measured contact pressure being indicative of whether there is skin contact.

The illustrated treatment device 2 may include an optional skin tone sensor 18 positioned on or in the head portion 6 that can be used to determine a skin tone of the skin that the head portion 6 is in contact with. The skin tone sensor 18 can measure a parameter that is indicative of the skin tone of the skin, and generate a measurement signal that comprises a time-series of measurements of the parameter. The measurement signal can be processed to determine the skin tone of the skin that the head portion 6 is in contact with. Typically, a skin tone sensor is used in a treatment device 2, particularly a photoepilator, to make sure that the light pulse has an intensity that is appropriate for the type of skin being treated, or even to prevent a light pulse being generated if the skin type is unsuitable for light pulses (e.g. darker skin which has a much higher melanin content).

In some embodiments the optional skin tone sensor 18 can be a light sensor and the parameter measured by the light sensor can be an intensity or level of light at a particular wavelength or multiple wavelengths reflected from the skin. The measured intensity or level of reflected light at a particular wavelength(s) can be indicative of the skin tone. The measured intensity or level of reflected light can be based on the concentration of melanin in the skin, and thus the measured intensity or level can indicate the melanin concentration. The melanin concentration can be derived, for example, from measurements of light reflection at 660nm (red) and 880nm (infrared) wavelengths.

The illustrated treatment device 2 also includes a user control 20 that can be operated by the user to activate the treatment device 2 so that the head portion 6 performs the required treatment operation on the body of the subject (e.g. the generation of one or more light pulses by the one or more light source(s) 12). The user control 20 may be in the form of a switch, a button, a touch pad, etc.

As noted above, one or more sensors may optionally be used to provide information on positions (including orientations) and/or movements of the treatment device over time so that suitable feedback can be determined, and in some embodiments the one or more sensors can be part of or be in the treatment device 2. In the case of the sensor(s) being one or more movement sensors, such as an accelerometer, gyroscope, etc., the one or more movement sensors can be part of, e.g. inside, the treatment device 2 (and as such are not shown in Fig. 1). In embodiments where the one or more sensors includes an imaging unit (e.g. a camera), the imaging unit may be on or in the treatment device 2. As shown in Fig. 1, an imaging unit 22 may be arranged on the housing 4 of the treatment device 2 so that it is able to obtain images or a video sequence of skin or a body part that is in front of the treatment device 2. That is, the imaging unit 22 is arranged on the housing 4 of the treatment device 2 so that it obtains images or a video sequence from a direction that is generally parallel to the direction in which light pulses are emitted by the light source(s) 12 through the aperture 10. In some implementations, the imaging unit 22 is arranged on the housing 4 such that, when the head portion 6 is placed into contact with the subject so that a light pulse can be applied to the part of the skin visible to the light source(s) 12 through the aperture 10, the imaging unit 22 is not able to view the part of the skin in contact with the aperture 10 and head end 6. In embodiments where the imaging unit 22 is arranged on or in the housing 4 of the treatment device 2, the imaging unit 22 is in a fixed arrangement with respect to the rest of the treatment device 2.

The treatment device 2 may further comprise an audio source 24 (sound generator), such as a loudspeaker, to issue a short audio pulse (also called flash sound) each time a light pulse (flash) is generated. This is particularly the case when light sources 12 are semiconductor source such as LEDs or VCSELs that do not generate the characteristic pulsed discharge flash sound of arc lamps. In other embodiments the flash sounds may be generated (e.g. automatically) by the light source when a light pulse is generated; for instance, a sound pulse like a tick sound may be generated each time a light pulse is emitted. The flash sound is e.g. a short audio pulse have a short duration (e.g. in the range of 0.1 to 2 s, such as approx. 1 s) at a certain frequency (e.g. 9 kHz or any other selected frequency) or frequency range (e.g. in the range of 1 to 10 kHz) and at an intensity that allows the user to hear the flash sound.

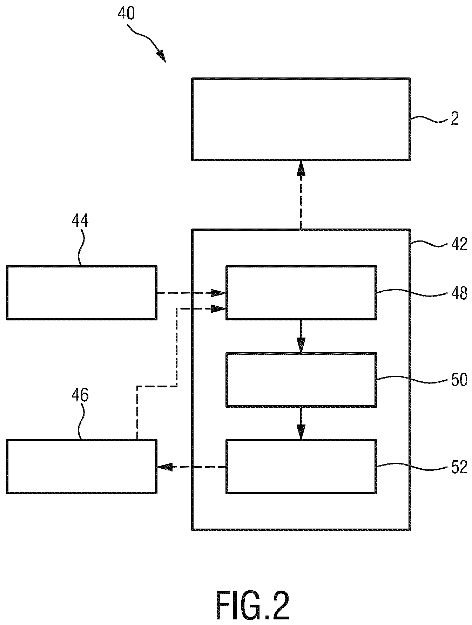

Fig. 2 is a schematic diagram of an exemplary system 40 according to the present invention comprising a treatment device 2 and an apparatus 42 for use with the treatment device 2, e.g. for controlling the treatment device 2 as e.g. shown in Fig. 1. The system 40 further comprises an audio sensor 44 (e.g. a microphone; there may also be multiple audio sensors) configured to sense an audio signal during a treatment operation, which may preferably be part of the apparatus 42, but may also be an external element. Further, the system 40 may optionally comprise a feedback unit 46 (e.g. a graphical user interface or sound output) for providing feedback to the user and/or subject, in particular regarding the progress of the treatment operation or any instructions to be followed by the user.

As noted above, in some embodiments the apparatus 42 can be a separate device to the treatment device 2, and thus the apparatus 42 may be in the form of an electronic device, such as a smart phone, smart watch, tablet, personal digital assistant (PDA), laptop, desktop computer, remote server, smart mirror, etc. In other embodiments, the apparatus 42, and particularly the functionality according to the invention provided by the apparatus 42, may be part of the treatment device 2.

The apparatus 42 comprises a processing unit 50 that generally controls the operation of the apparatus 42 and enables the apparatus 42 to perform the method and techniques described herein. The processing unit 50 may further be configured to receive a measurement signal from the audio sensor 44, process the measurement signal to determine the feedback to be provided about the treatment operation, and provide a feedback control signal to the feedback unit 46. Thus the processing unit 50 may be configured to receive the measurement signal from the audio sensor 44 either directly in embodiments where the sensor 44 is part of the apparatus 42, or via another component in embodiments where the sensor 44 is separate from the apparatus 42. In either case, the processing unit 50 can include or comprise one or more input ports or wires for receiving the measurement signal via the input unit 48. The processing unit 50 can also include or comprise one or more output ports or wires for outputting the feedback control signal via the output unit 52.

The processing unit 50 can be implemented in numerous ways, with software and/or hardware, to perform the various functions described herein. The processing unit 50 may comprise one or more microprocessors or digital signal processors (DSPs) that may be programmed using software or computer program code to perform the required functions and/or to control components of the processing unit 50 to effect the required functions. The processing unit 50 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor) and/or hardware.

The processing unit 50 may optionally comprise or be associated with a memory unit (not shown). The memory unit can store data, information and/or signals (including image(s)) for use by the processing unit 50 in controlling the operation of the apparatus 42 and/or in executing or performing the methods described herein. In some implementations the memory unit stores computer-readable code that can be executed by the processing unit 50 so that the processing unit 50 performs one or more functions, including the methods described herein. In particular embodiments, the program code can be in the form of an application for a smart phone, tablet, laptop, computer or server. The memory unit can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment shown in Fig. 2, as the apparatus 42 is shown as being separate from the sensor(s) 44, the apparatus 42 may include an input unit 48, such as interface circuitry, to enable the apparatus 42 to receive the measurement signal(s) from the sensor(s) 44. The interface circuitry 48 in the apparatus 42 enables a data connection to and/or data exchange with other devices, including any one or more of the sensor(s) 44, the treatment device 2, servers and databases. The connection to the sensor(s) 44 (or any electronic device, such as treatment device 2) may be direct or indirect (e.g. via the Internet), and thus the interface circuitry 48 can enable a connection between the apparatus 42 and a network, or directly between the apparatus 42 and another device (such as sensor(s) 44 and/or treatment device 2), via any desirable wired or wireless communication protocol. For example, the interface circuitry 48 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 48 (and thus apparatus 42) may include one or more suitable antennas for transmitting/receiving over a transmission medium (e.g. the air). Alternatively, in the case of a wireless connection, the interface circuitry 48 may include means (e.g. a connector or plug) to enable the interface circuitry 48 to be connected to one or more suitable antennas external to the apparatus 42 for transmitting/receiving over a transmission medium (e.g. the air). The interface circuitry 50 is connected to the processing unit 50.

Further, the apparatus 42 may comprise an output unit 52, such as output circuitry to enable the apparatus to output information, in particular to the feedback unit 46. The output unit can generally be configured and have similar or the same functions as the input unit 48.

The feedback unit 46 may include one or more components that enables a user of apparatus 42 to input information, data and/or commands into the apparatus 42 (e.g. via the input unit 48), and/or enables the apparatus 42 to output information or data to the user of the apparatus 42 (via the output unit 52). The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

In some embodiments, the feedback unit 46 is attached to or part of the treatment device 2. In other embodiments, the feedback unit 46 is part of the apparatus 42. In these embodiments the feedback unit 46 may be part of, or make use of, any of the output components of user interface components of the apparatus 42. In further embodiments, the feedback unit 46 can be separate from the treatment device 2 and the apparatus 42.

It will be appreciated that a practical implementation of an apparatus 42 may include additional components to those shown in Fig. 2. For example, the apparatus 42 may also include a power supply, such as a battery, or components for enabling the apparatus 42 to be connected to a mains power supply.

The present invention has different aspects, in particular the aspect of actually using the apparatus 42 during an actual treatment operation and the aspect of training the apparatus 42 to reliably recognize flash sounds emitted by the treatment device 2 during the treatment operation.

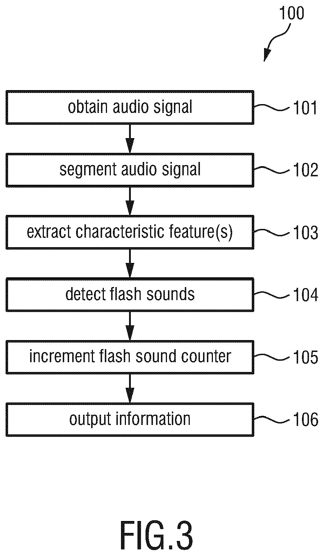

A flowchart of an embodiment of a method 100 according to the first aspect, which may be used during actual operation of the treatment device, is illustrated in Fig. 3. In a first step 101 of the method 100 an audio signal sensed during a treatment operation (by the audio sensor 44) is obtained (by the input unit 48), i.e. received or retrieved from the audio sensor 44. The obtained audio signal is segmented into audio segments, in particular into audio segments of a set or predetermined duration (e.g. of 1 s or any other suitable duration), in a second step 102. In another (combinable) embodiment, audio segments of said predetermined duration are directly obtained by the input unit 48 during the treatment operation. Per audio segment, one or more characteristic features (as explained in more detail below) are extracted from the audio segments in a third step 103. Then, in a fourth step 104, per audio segment, it is detected if the audio segment comprises a flash sound. This is done by applying a trained algorithm or computing system that has been trained on a plurality of audio segments each including one or more of the characteristic features to detect flash sounds in the audio segments. In a fifth step 105, a flash sound counter counting the number of detected flash sounds since start of a treatment operation or a set or user-instructed moment is increased each time a flash sound has been detected. Finally, in step 106, information is outputted via the output unit 52 to the feedback unit 46. The information may include one or more of the flash sound counter (i.e. its current value), an indication if the flash sound counter exceeds a flash sound threshold, and an indication that the treatment operation has finished.

The flash sound threshold may e.g. be preset and reflect a maximum number of flashes required or appropriate or prescribed for a certain body region to be treated. Such a setting may be stored in the apparatus 42 or may be inputted by the user, who may take it from a table e.g. in the user manual or may select or compute it either manually or under support by user guidance on the feedback unit or the apparatus 42 or the treatment device 2. E.g., the flash sound threshold may not only depend on the size of the treatment area, but also on the type of skin, the color of skin, the number of previous treatments, the time since last treatment, etc.

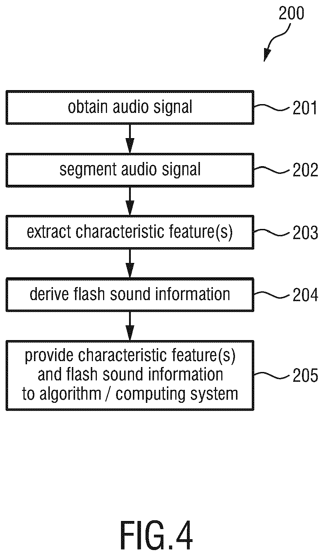

A flowchart of an embodiment of a method 200 according to the second aspect, which may be used for training, is illustrated in Fig. 4. In a first step 201 of the method 200 an audio signal sensed (by the audio sensor 44) during a treatment operation or artificially created (e.g. by a sound processor of the apparatus or an external device) is obtained. The audio signal thus is or simulates a "real" audio signal as it would be recorded during a treatment session and thus includes multiple flash sounds (and optionally background noise). It may thus have a length of several seconds or tens of seconds or even some minutes. Further, the audio signal comprises an annotation, which may be generated by a user while listening to the audio signal or may be generated automatically by an algorithm or the device that artificially created the audio signal (and simultaneously generated the annotation). The annotation of the audio signal comprises flash sound information indicating flash sounds within the audio signal, in particular temporal information of flash sounds, such a beginning, end, highest peak, duration, etc. of the flash sound.

The (annotated) audio signal is segmented into audio segments, in particular into audio segments of a set or predetermined duration, in a second step 202. In a third step 203, per audio segment, one or more characteristic features, e.g. a set of MFCC coefficients (as will be explained below in more detail), are extracted from the audio segments. In a fourth step 204, from the annotation of the audio signal, flash sound information indicating if an audio segment comprises a flash sound is derived per audio segment, e.g. flash sound information is read from a file or metadata representing the annotation associated with or embedded in the (annotated) audio signal. In a fifth step 205, per audio segment, the extracted one or more characteristic features and the derived flash sound information are provided to an algorithm or computing system to train it for the detection of a flash sound within an audio segment based on one or more characteristic features.

Several machine learning and deep learning architectures are generally known for audio classification. One such use case is the UrbanSound8K, which is a public dataset. This dataset contains 8732 labeled sound extract of urban sound of ten classes: car horn, dog bark gun shot, children playing, air conditioner, drilling, vehicle engine running, jack hammer, siren, and street music. Using mel-frequency cepstral coefficients (MFCCs), feature extraction and applying Zero padding over it, this input is passed to Convolutional Neural Network (CNN) model. Using MFCC features a training accuracy of 98.12% and validation accuracy of 91.23% is achieved. For improving the accuracy feature padding rather than zero padding is applied and other features like zero-crossing rate, pitch, RMSE, chroma along with MFCC is used. After training for 100 epochs a training accuracy of 99.21% and validation accuracy of 97.29% is achieved.

Another dataset is ESC-50 which consists of fifty classes. The dataset consists of 2000 audio pieces each with 5 seconds duration. Performance on ESC-50 is 94% accuracy. Using audio spectrogram transformer, a convolution-free architecture is availed audio classification. A training accuracy of 94.7% is achieved using audio spectrogram transformer.

TUT Rare Sound Events 2017 consists of isolated sound events for each target class and recordings of everyday acoustic scenes to serve as background. The target sound event categories are baby crying, glass breaking, gun shot. Several events were organized for solving the above challenge and a best accuracy of ~93% on evaluation dataset and ~96 % on development dataset was achieved. The approach proposed for solving this problem is divided in 4 steps: Feature extraction (log-magnitude and mel spectrogram), converting spectral feature with 1D convNet, Temporal Dependency with RNN-LSTM, and detecting the presence and onset of time of audio event with post processing.

Uyen Diep (Sally), "Classification of Cough Events", ECE Senior Capstone Project 2017, Tech Notes discloses cough analysis as part of an automated cough monitoring system for patients, that is divided into two main stages, event detection and event classification. In this method, sound events are broken up into overlapped time frames of 32ms length or 16ms length. The features of each frame are calculated and fed into a classifier to determine if the frame has characteristic of a cough. The results are then combined to make an overall decision for the event. MFCC may be used in this method.

The known approaches for audio classification based on signal processing are mainly multiclass classification using deep neural network. The sounds that are mainly being classified persists for few seconds and are continuous in nature. In contrast, in the flash detection in the present scenario of photoepilation, flashes generally persist of few milliseconds and are aperiodic. In an embodiment, a classical machine learning approach for building a binary classification model to detect whether the flash is present or not is being used for flash sound detection and training. The significance of using such a machine learning (ML) approach over deep learning is the optimized size of the model that provides an easy in app deployment of the model.

While doing hair removal treatment, e.g. at home, there are multiple challenges for quality hair removal treatment, proper recommendation, and frictionless experience. There is particularly a need to detect the sound pattern of the treatment device. The sound pattern signal consists of flash sounds that are emitted during the usage of the treatment device, but there can be potential background noise such as TV sound, radio, baby crying, traffic noise, people communicating, a door being opened or closed, etc. Hence, there is a need to accurately identify the intended sound pattern within the audio signal. The present invention employs signal processing and a trained algorithm or computing system, in particular machine learning (ML), together with an appropriate set of characteristic features (e.g. 12-13 selected MFCC features) to reliably and accurately detect flash sounds despite any background noise. The trained algorithm or computing system, e.g. an ML model (such as XGBoost) learns in the training process to distinguish between the background noise (non-flashes) to that of a flash sound. Hence, the annotation helps the model to understand the pattern, which comprises flash and no-flash audio segments.

The present invention thus addresses a number of challenges including one or more of:

- Ability to accurately detect sound with varying amplitude based on audio captured from distance of 50 cm to 1 m
- Robust to various background noises such as TV, door opening or closing, running tap water, radio, etc.
- Auto detection of accurate number of flashes (being used during a treatment)
- Approach to reduce annotation effort
- Segregating various background noises (such as TV, water dropping, etc.) overlapping the flash sound
- Accurate detection to process the signals and detect only the flash sound
- Ability to have lightweight and low-latency architecture for integration of an artificial intelligence (AI) algorithm in both conventional applications and operating systems

Contrary to prior art approaches like the cough detection method disclosed by Uyen Diep in the document cited above, the present invention enables to reliably detect the (artificial) flash sounds issued by treatment device in the presence of background noise, because the flash sound characteristics are very different to the characteristics of human coughs. The problem solved by the present invention can be regarded as detecting the accurate number of flash sounds occurring during a treatment. The flash sounds are not continuous in nature and persist for some fraction of seconds, i.e. a flash sound is aperiodic and transient in nature (in contrast to aperiodic continuous sounds and periodic (simple or complex) sounds). Raw audio signals acquired during a treatment of different body areas of subjects like leg and armpit have different durations. For instance, the armpit audio length varies from 30 seconds to 105 seconds and the leg audio length varies from 30 seconds to 180 seconds. The audio signals comprise flash sounds with a minimum interval of e.g. 0.8 seconds. The audio signals also comprise background noise.

As a result, the processing approach of the present invention is different from prior art. For example, while sound events like coughs can be grouped into overlapping frames, in order to count the exact number of flashes, the present approach divides the audio signal into non-overlapping audio segments during preprocessing. The non-overlapping audio segments are especially useful for avoiding double counting of flashes. MFCC systems usually use only 8-13 cepstral coefficients. The zeroth coefficient typically used in prior art disclosed e.g. by Uyen Diep represents the average log-energy of the input signal and only carries little speaker-specific information. It is not used in preferred embodiments of the present invention as it will lead to detecting background noise. The present invention thus provides the ability to detect low amplitude, short duration pulses that is produced by the treatment device with varied background noise. MFCC is just a feature extraction step, the right combination of these features and appropriate algorithm / computing system (e.g. the appropriate ML model) contribute to achieving the desired effects and solving the addressed problem, in particular to provide low latency, low memory footprint and high accuracy because of its ability to distinguish subtle flash sound from varying background noise due to appropriate feature selection.

In an exemplary implementation, the data (audio signal) is collected by a microphone of a smartphone at a close distance (e.g. in the range of 0.5 to 2.5 m) from the treatment device. The audio signal comprises flash sounds along with any existing background noise.

During training, an annotation may be performed on the raw audio signal wherever flash sounds are present, i.e., an indication, such as a comment or metadata, is added to the audio signal indicating the time moments when flash sounds are present in the audio signal. A preprocessing of the audio signal is performed by quantizing the raw audio signal or the annotated signal into segments of a set duration e.g. one second chunks and labeling the segments as ticks (i.e. flash sounds) and non-ticks (non-flash sounds). Furthermore, background noise filtering may be performed and time and frequency domain features such as MFCC, Mel-spectrogram etc., may be extracted from the audio signal, thus creating an audio feature array. In an embodiment a ML model is built by using the feature array, which has an ability to detect the flash sounds in near real time. The ML model may e.g. be deployed in an app (application or software program) as a program file, which enables the model to run on the desired device, such as a smartphone.

While generally any algorithm or computing system can be used, and generally any kind of a learning system, machine learning, classification model, binary classification model, neural network, or convolutional neural network may be used as trained algorithm or computing system, in an embodiment an XGBoost binary classification model is built to detect whether a flash sound is present in an audio signal or not. How to build such an XGBoost classifier is generally known in the art. An exemplary XGBoost classifier may be built with the following hyper parameters: learning_rate=0.02, n_estimators=600, objective='binary:logistic'. Other parameters and other classifiers or ML models may be used as well. In this context it shall be noted that, generally, the particular implementation or architecture or layout of the trained algorithm or computing system is not essential, but e.g. a standard learning system or neural network with known or conventional architecture may be used for the task of detecting flash sounds.

For the training (as illustrated in Fig. 4) annotations of the audio signals are used. An embodiment how such annotations are created will be described in the following. In the implementation described above, audio data is collected, e.g. in real time or with temporary storage, by the microphone of a smartphone, preferably at a distance of 0.5 m to 2.5 m from the photoepilation device. The flash sounds are extracted by the ML model. The extracted data may comprise start and end time of each flash sound in the audio file. Generally, any annotation tool by which annotation can be added to an audio file may be used for this purpose. In an exemplary implementation the annotation tool "Barista" of the applicant is used.

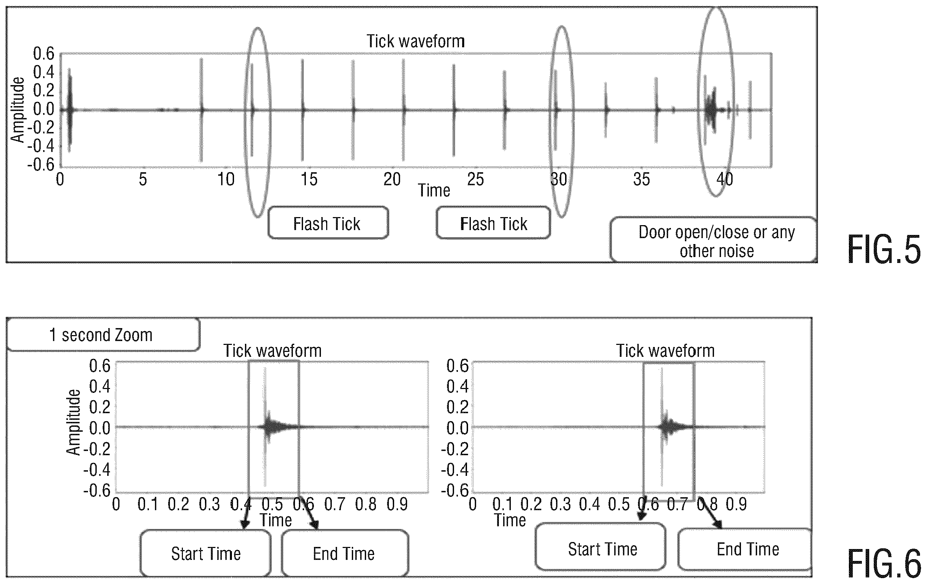

In an embodiment the raw audio file is opened, peaks are visualized and it is listened to audio (e.g. only once). Such a visualization of part of an audio file is shown in Fig. 5, in which flash sounds ("Flash Tick") and background noise ("Door open/close or any other noise") are indicated. Playing the audio file is then started, which is paused where the flash sound can be visualized or heard. It may then be zoomed in on that area. Fig. 6 shows such a visualization with a one second zoom on two flash sounds, in which start time and end time are indicated. Then, the area to be annotated can be selected. The selected area may be played and listened to for cross verification. This process may be repeated until the end of audio file.

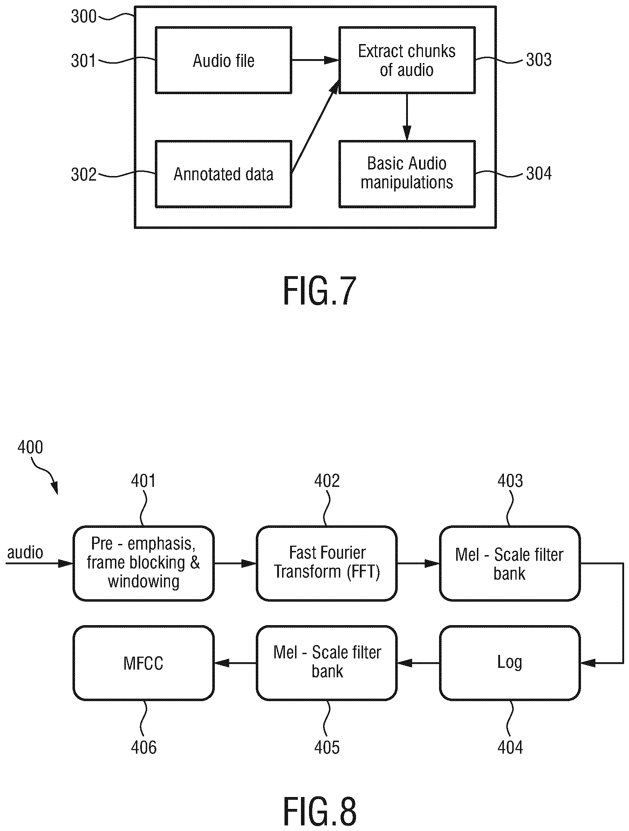

In an embodiment, as schematically illustrated in Fig. 7, audio pre-processing 300 of a raw audio file 301 and/or an annotated audio file 302 (with indications of flash sound) may be performed by quantizing the audio file into audio segments (chunks) 303 of a certain length (e.g. in the range of 0.1 to 5 s, e.g. 0.5 or 1 s). Each audio segment may then be labeled as flash sound or non-flash sound. Further, basic audio manipulations 304 may be made, such as repeating the pre-processing a number of times, changing the pitch of the audio signal of the audio file, etc. This is done since the raw audio signal may not always have sufficient or optimal quality, e.g. may be corrupted with background noise.

In an embodiment, as schematically illustrated in Fig. 8, feature extraction 400 is performed for analyzing the audio data. The audio data may generally be regarded as a 3D signal which represents time, amplitude and frequency. Since the 3D format cannot (or not easily) be directly processed by AI models, the feature extraction approach is availed. Before passing the audio signals to an XGBoost classification model, the signals are converted into an array format which would then be ready for further processing and analysis by an AI model. Generally, in a first step 401 pre-emphasis, frame blocking and windowing are performed, before an FFT is applied in a second step 402. Subsequently, a filter bank 403, e.g. a Mel-Scale filter bank, a logarithm 404 and again the filter bank 405 are applied to obtain the desired features, e.g. MFCC features 406.

MFCC refers to mel scale which is a perceptually relevant scale for pitch. From the MFCC feature a number of coefficient values are obtained that describe the characteristic of an audio segment, i.e. a piece of sound. In an embodiment, in the first step 401 the given audio signals are divided into short frames (audio segments). This step is performed since frequencies in a signal change overtime; calculating the Fourier transform across the entire audio signal would thus lead to loss in the frequency contours of the audio signal over time. Further, windowing (e.g. using a Hamming window) is done to cross check that the data is infinite and to reduce spectral leakage.

In the second step 402, the discrete Fourier transform is calculated to get a simplified version of Fourier transform and to get real-valued coefficients (e.g. in total 39 coefficients per frame). In the third step 403 mel spaced filter banks are applied which includes to convert lowest and highest frequency to mel, to create equally spaced points as the number of mel bands that shall be used, to convert points back to Hertz, to round these points to nearest frequency bins (because there are discrete signals) and to create triangular filters. As a result, the 39 coefficients of the MFCC feature are obtained.

Generally, every audio signal comprises multiple features. However, the characteristics that are relevant to the problem that shall be solved need to be extracted. Using these audio features, an intelligent audio classification model can be built and trained.

In an embodiment of the present invention Mel frequency cepstral coefficients MFCCs) are used as characteristics. The MFCCs of a signal are a small set of features (usually about 10-20) which concisely describe the overall shape of a spectral envelope. MFCC models the characteristics of human voice. There are generally around 39 coefficients per frame. In an embodiment, only the first coefficients, e.g. the first 12-13 coefficients (preferably the first 13 coefficient) not including the zeroth component that represents the average log-energy of the input signal, are used since the first few coefficients encode the majority of the information (e.g. spectral envelope, formants, etc.). The MFCC feature works well in music and speech processing but lacks robustness to noise.

There are multiple audio categorizations based on the categories indicated in the following table:

| Audio Categorization | Types | Description |
|---|---|---|
| Level of abstraction | High | Instrumentation, key chords, melody, rhythm |
| | Medium | Pitch and best related descriptors such as note offsets, MFCCs |
| | Low | Energy, spectral centroid, spectral flux, Zero crossing Rate |
| Temporal scope | Instantaneous (~50ms) | Feature that give us instantaneous information about the audio signal. This considers very short chunk of audio signals, which usually stays around 50 to 100 ms |
| | Segment | Audio features that are calculated over segments ranges from 10 or 15 seconds. |
| | Global | Feature that provide aggregated information about entire audio file. |
| Music aspect | | Audio features that are being classified on the basis of musical aspect that they give glimpse of such as beat, timbre, pitch, harmony, etc. |
| Signal domain | Time domain | Extracted from waveform, x-axis time, y-axis amplitude (Zero crossing Rate, RMSE energy, amplitude envelope) |
| | Frequency domain | Sound is characterized by frequency. Time domain does not have any. Other feature comes under frequency domain (spectral centroid, spectral flux, Band Energy Ratio ) |
| | Time-frequency domain | Spectrogram, Mel spectrogram, Constant Q transform Spectrogram - start with Time domain and apply short time Fourier transform |
| ML approach | Traditional ML | Zero crossing Rate, RMSE energy, amplitude envelope, spectral centroid, spectral flux, Band Energy Ratio, Spectral Roll off, spectral spread |
| | Deep learning | Spectrogram, raw audio |

Audio feature categorization can be based on the following domains:

| Signal Domain | Time Domain | Zero Crossing Rate | $$zcr = \frac{1}{T-1} \sum_{t=1}^{T-1} 1_{\mathbb{R}_{<0}}\left(s_t s_{t-1}\right)$$ |
| | | RMSE Energy | $$RMS(t) = \sqrt{\frac{1}{T} \sum_{i=1}^{T} w_i(t) x_i^2(t)}$$ |
| | | Amplitude Envelope | $$AE_t = \max_{k=t \cdot K}^{(t+1) \cdot K - 1} s(k)$$ |
| | Frequency Domain | Spectral Centroid | $$SC_t = \frac{\sum_{n=1}^{N} m_t(n) \cdot n}{\sum_{n=1}^{N} m_t(n)}$$ |
| | | Band Energy Ratio | $$BER_t = \frac{\sum_{n=1}^{F-1} m_t(n)^2}{\sum_{n=F}^{N} m_t(n)^2}$$ |
| | Time-frequency Domain | Spectrogram | A spectrogram is a visual representation of the spectrum of frequencies of a signal as it varies with time. |
| | | Constant Q transform | $$\delta f_k = 2^{1/n} \cdot \delta f_{k-1} = (2^{1/n})^k \cdot \delta f_{min}$$ |

After creating the audio segments in the pre-processing step, the extracted MFCC features are passed over to the model.

In the following, an embodiment of AI model training is described in more detail. The input to the model may be a matrix (or vector) with e.g. 13 columns as MFCC coefficients and the number of rows equivalent to the length of the audio file (e.g. for each audio segment of e.g. 1 s length there is one row).

In an embodiment the following 13 MFCC coefficients are used for each audio segment of 1 s length (i.e., these 13 MFCC coefficients represent the 13 columns of a matrix):

    mfcc_second_derivative_std_1
    mfcc_second_derivative_std_2
    mfcc_second_derivative_std_3
    mfcc_second_derivative_std_4
    mfcc_second_derivative_std_5
    mfcc_second_derivative_std_6
    mfcc_second_derivative_std_7
    mfcc_second_derivative_std_8
    mfcc_second_derivative_std_9
    mfcc_second_derivative_std_10
    mfcc_second_derivative_std_11
    mfcc_second_derivative_std_12
    mfcc_second_derivative_std_13.

It shall be noted that these are common names generally known to the skilled person; these features are selected and ranked based on their predictive power to identify the flash sound. Here, the coefficient "mfcc_second_derivative_std_1" is the MFCC second derivative standard deviation (also called as delta - delta coefficient). To arrive at the second derivative, MFCC features are extracted and then the second derivative over those MFCC features is computed.

The delta MFCC is computed per frame. For each frame, it is the current MFCC values minus the previous MFCC frame values. It is as good as applying a smoothing filter, as the difference operation is sensitive to noise, leading to better accuracy. These matrix coefficients are then input to the model along with the target feature (during training).

Exemplary values for the 13 coefficients for an exemplary audio segment of 1 s length are: 2.17, 2.14, 1.34, 1.60, 1.94, 1.71, 1.62, 1.67, 1.66, 1.24, 1.48, 1.66, 1.65.

An exemplary matrix looks as follows (wherein the first 13 columns are the feature vector (X) and the last column (Tick) is the target variable (Y), and each row represents the values for different audio segments):

| nd_deriva | nd_deriva | nd_deriva | nd_deriva | nd_deriva | nd_deriva | nd_deriva | nd_deriva | nd_deriva | nd_deriva | nd_deriva | nd_deriva | d_deriva | Tick |
|---|---|---|---|---|---|---|---|---|---|---|---|---|---|
| 2.167597 | 2.142322 | 1.335738 | 1.598709 | 1.937927 | 1.710204 | 1.624925 | 1.671166 | 1.658527 | 1.240797 | 1.481133 | 1.662306 | 1.654 | 0 |
| 5.092344 | 4.555452 | 2.168352 | 2.302918 | 2.063551 | 1.41976 | 1.631213 | 2.324844 | 1.912578 | 1.522205 | 1.654556 | 2.033623 | 1.401 | 1 |
| 1.350836 | 1.571874 | 1.548549 | 1.265196 | 1.481218 | 1.448998 | 2.002048 | 1.745927 | 1.323804 | 1.401121 | 1.780856 | 1.850187 | 1.507 | 0 |
| 15.37377 | 3.162368 | 3.534241 | 5.272236 | 2.301794 | 3.599918 | 2.47849 | 2.958104 | 2.312761 | 2.507938 | 2.137188 | 2.008357 | 1.838 | 1 |

An exemplary model used is an XGBoost classifier to classify whether a flash sound is present or not in each audio segment of e.g. 1 s length based on MFCC feature extracted. In general, an ensemble model performs better in classification in regression tasks than their non-ensemble counterparts such as linear regression, logistic regression etc. XGBoost is an ensemble Machine Learning algorithm that uses decision-tree and gradient boosting framework. It can be used to solve multiple regression, classification, ranking, and user-defined prediction problems. Thus, the matrix (the rows with the values for the audio segments) which is the output of MFCC computation, is fed to the ML model for classification. During training each frame, which is annotated as flash or not flash is passed along with the feature matrix as target variable. While generally different coefficients (i.e. characteristics) may generally be used between training and actual use, it is preferred that the same coefficients are used for training and actual use of the device and method.

Audio files comprise treatment sound including flash sound masks with device fan noise along with other background noise. Collected audio files of many treatments, which may be used for training the AI model and others for validation purpose. Armpit audio length varies from 30 seconds to 105 seconds and leg audio length varies from 30 seconds to 180 seconds. A training accuracy 96% and validation accuracy 90% is achieved using the above algorithm.

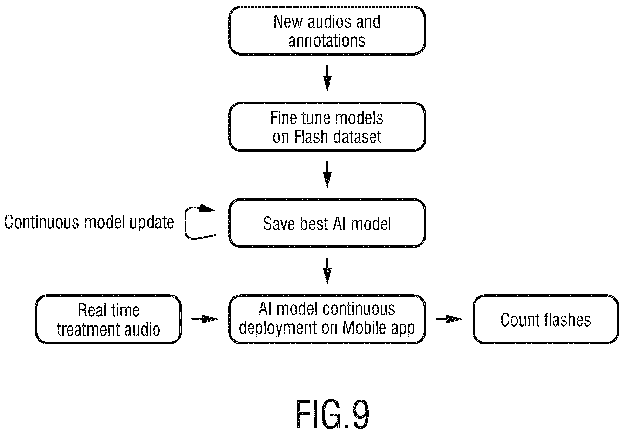

A flash detection model may be deployed in different operating systems, such as iOS, Android, etc. Fig. 9 illustrates a flowchart of the process from model deployment to production. Considering the base model retraining the model on new audios is included as important step. Using new datasets fine tuning of the model and its efficacy and saving the best AI model is foreseen. The model update is an iterative process comprising continuous or repeated retraining and fine tuning of the model at its best and deploying the same in a mobile application.

In the production phase, by using the microphone of a smartphone (or other useful device) real time audio is taken as input, which is passed over to the latest model to detect flashes.

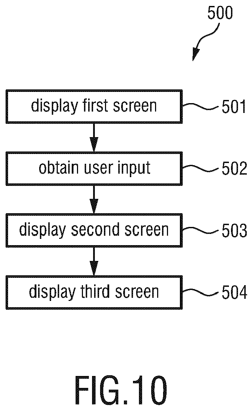

A flowchart of an embodiment of a method 500 according to the third aspect, which may be used for providing a user interface, is illustrated in Fig. 10. The method 500 is generally configured to control a user interface to display, in a first step 501, a first screen requesting user input regarding a treatment operation. In a second step 502, user input is obtained via the first screen, the user input comprising an image of a body part of a subject to be treated and/or size information of the size of the body part of the subject to be treated. In a third step 503, a second screen indicating connectivity of the apparatus or an electronic device comprising the apparatus with the treatment device is displayed. In a fourth step 504, a third screen is displayed indicating at least one of:

- an operation status of the skin treatment device,
- a flash sound counter counting the number of detected flash sounds since start of a treatment operation or a set or user-instructed moment,
- an indication if the flash sound counter exceeds a flash sound threshold, and
- an indication that the treatment operation has finished.

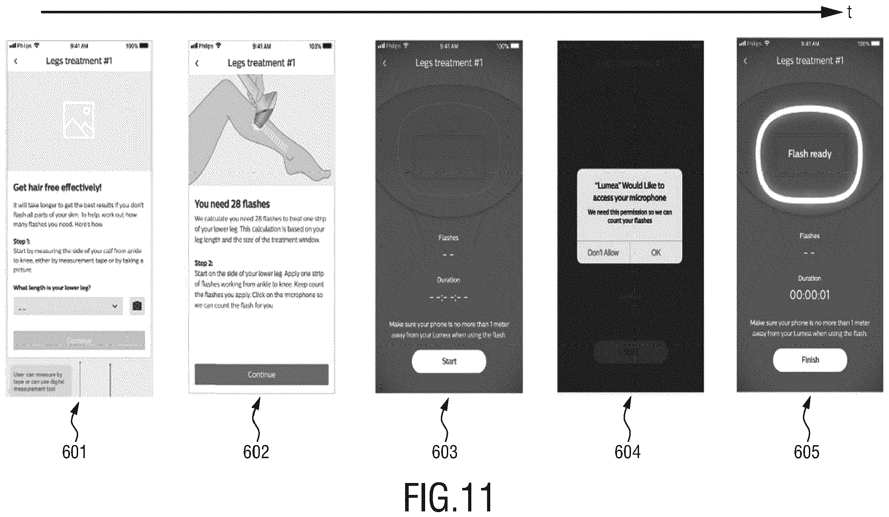

A corresponding user interface is illustrated in different phases in Fig. 11. The screen 601 (representing the first screen in step 501) shows a user guide of how to effectively take the treatment and will ask the user for size of the desired treatment area, e.g. leg length. The screen 602 shows once the user enters the leg length (in step 502); some ideal number of flashes will be displayed for proper treatment. After clicking on continue, the user will land on screen 603 where it will ask to click the start button to start the treatment. Starting the treatment will ask on screen 604 (representing the second screen in step 503) for microphone access (or, more generally, to indicate connectivity). Once it is granted detecting the flash sounds will be started as displayed on screen 605, and the total number of flashes that have been used during the entire treatment will be incremented as displayed on screen 5 (representing the third screen in step 504). In another embodiment the remaining number of flashes required for treating the desired treatment area may be displayed, or both the total number of applied flashes and the remaining number of flashes may be displayed.

The screens shown in Fig. 11 are to be understood as examples. Other information may be displayed on each screen as well, and there may be other designs and numbers of screens.

The present invention provides the advantage of automating the flash count per treatment which will help the user with the counting for subsequent treatments. A treatment may typically take around 30 - 40 flashes. Currently, the user has to keep track of flash counts manually which leads to poor user experience. Thus, a proper recommendation of the device will remove the friction in usage of the device (i.e., automating the counting of the flashes without the need of the user to manually count during the treatment) and lead to better user experience and satisfaction. The presented method has been tested with different background noises and models that have the ability to identify the flash sound. Whenever the treatment is not meeting the expectation levels, it can be checked whether the guidance or recommendation has been followed or not.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. An apparatus (42) for use with a treatment device (2) configured to perform a treatment operation on a body part of a subject, wherein the treatment device is configured to apply light pulses to skin of the body part to perform the treatment operation and to output a flash sound per light pulse, the apparatus (42) comprising

   - an input (48) configured to obtain an audio signal sensed during a treatment operation;
   - a processing unit (50) configured to

      - segment the audio signal into audio segments, in particular into audio segments of a set or predetermined duration,
      - extract, per audio segment, one or more characteristic features from the audio segments,
      - detect, per audio segment, if the audio segment comprises a flash sound by applying a trained algorithm or computing system that has been trained on a plurality of audio segments each including one or more of the characteristic features to detect flash sounds in the audio segments, and
      - change a flash sound counter counting the number of detected flash sounds since start of a treatment operation or a set or user-instructed moment; and

   - an output (52) configured to output one or more of:

      - the flash sound counter,
      - an indication if the flash sound counter exceeds a flash sound threshold, and
      - an indication that the treatment operation has finished.

2. Apparatus as claimed in claim 1,
   wherein the processing unit (50) is configured to use a learning system, machine learning, a classification model, a binary classification model, a neural network, or a convolutional neural network as trained algorithm or computing system.

3. Apparatus as claimed in any one of the preceding claims,
   wherein the processing unit (50) is configured to extract one or more features related to time, amplitude and frequency of the audio segment as characteristic feature.

4. Apparatus as claimed in claim 3,
   wherein the processing unit (50) is configured to extract, as characteristic feature of an audio segment, one or more of start time, end time, amplitude, gradient, instrumentation, key chords, melody, rhythm, pitch, energy, note offset, one or more mel-frequency cepstral coefficients (MFCCs), mel spectrogram, spectral centroid, spectral flux, zero crossing rate, beat, timbre, pitch, harmony, root-mean-square energy, amplitude envelope, band energy ratio, spectrogram, constant Q transform, spectral roll off, and spectral spread.

5. Apparatus as claimed in claim 3 or 4,
wherein the processing unit (50) is configured to arrange the extracted features related to time, amplitude and frequency of the audio segment into an array and to subject the array to the trained algorithm or computing system to detect, per audio segment, if the audio segment comprises a flash sound.

6. Apparatus as claimed in any one of the preceding claims,
wherein applying the trained algorithm or computing system onto an audio segment by the processing unit (50) includes building classification boundaries based on the one or more characteristic features.

7. Apparatus as claimed in any one of the preceding claims,
wherein the processing unit (50) is configured to use the result of the detection for further training of the algorithm or computing system.

8. Apparatus, in particular as claimed in any one of the preceding claims, for use with a treatment device (2) configured to perform a treatment operation on a body part of a subject, wherein the treatment device is configured to apply light pulses to skin of the body part to perform the treatment operation and to output a flash sound per light pulse, the apparatus (42) comprising

    - an input (48) configured to obtain an audio signal sensed during a treatment operation or artificially created;
    - a processing unit (50) configured to

        - segment the audio signal into audio segments, in particular into audio segments of a set or predetermined duration,
        - extract, per audio segment, one or more characteristic features from the audio segments,
        - derive, per audio segment, from an annotation of the audio signal flash sound information indicating if an audio segment comprises a flash sound, the annotation of the audio signal indicating flash sounds within the audio signal, in particular temporal information of flash sounds, and
        - provide, per audio segment, the extracted one or more characteristic features and the derived flash sound information to an algorithm or computing system to train it for the detection of a flash sound within an audio segment based on one or more characteristic features.

9. Apparatus as claimed in claim 8,

    wherein the input (48) is configured to obtain the annotation of the audio signal and/or
    wherein the processing unit (50) is configured to generate the annotation of the audio signal, in particular automatically or based on user input.

10. Apparatus, in particular as claimed in any one of the preceding claims, for use with a treatment device (2) configured to perform a treatment operation on a body part of a subject, wherein the treatment device is configured to apply light pulses to skin of the body part to perform the treatment operation and to output a flash sound per light pulse, the apparatus (42) comprising an output (52) configured to control a user interface to:

    - display a first screen requesting user input regarding a treatment operation;
    - obtain user input via the first screen, the user input comprising an image of a body part of a subject to be treated and/or size information of the size of the body part of the subject to be treated;
    - display a second screen indicating connectivity of the apparatus or an electronic device comprising the apparatus with the treatment device;
    - display a third screen indicating at least one of:

        - an operation status of the skin treatment device,
        - a flash sound counter counting the number of detected flash sounds since start of a treatment operation or a set or user-instructed moment,
        - an indication if the flash sound counter exceeds a flash sound threshold, and
        - an indication that the treatment operation has finished.

**11.** A system, comprising:

a treatment device (2) comprising one or more light sources (14, 16) configured to generate light pulses for application to skin of a subject's body to perform a treatment operation, wherein the treatment device (2) is configured to generate a flash sound per light pulse;
an audio sensor (44) configured to sense an audio signal during a treatment operation; and
an apparatus (42) as claimed in any one of the preceding claims.

**12.** A method for use with a treatment device (2) configured to perform a treatment operation on a body part of a subject, wherein the treatment device is configured to apply light pulses to skin of the body part to perform the treatment operation and to output a flash sound per light pulse, the method comprising

- obtaining an audio signal sensed during a treatment operation,
- segmenting the audio signal into audio segments, in particular into audio segments of a set or predetermined duration,
- extracting, per audio segment, one or more characteristic features from the audio segments,
- detecting, per audio segment, if the audio segment comprises a flash sound by applying a trained algorithm or computing system that has been trained on a plurality of audio segments each including one or more of the characteristic features to detect flash sounds in the audio segments, and
- changing a flash sound counter counting the number of detected flash sounds since start of a treatment operation or a set or user-instructed moment; and
- outputting one or more of:

- the flash sound counter,
- an indication if the flash sound counter exceeds a flash sound threshold, and
- an indication that the treatment operation has finished.

**13.** A method, in particular as claimed in claim 12, for use with a treatment device (2) configured to perform a treatment operation on a body part of a subject, wherein the treatment device is configured to apply light pulses to skin of the body part to perform the treatment operation and to output a flash sound per light pulse, the method comprising

- obtaining an audio signal sensed during a treatment operation or artificially created,
- segmenting the audio signal into audio segments, in particular into audio segments of a set or predetermined duration,
- extracting, per audio segment, one or more characteristic features from the audio segments,
- deriving, per audio segment, from an annotation of the audio signal flash sound information indicating if an audio segment comprises a flash sound, the annotation of the audio signal indicating flash sounds within the audio signal, in particular temporal information of flash sounds, and
- providing, per audio segment, the extracted one or more characteristic features and the derived flash sound information to an algorithm or computing system to train it for the detection of a flash sound within an audio segment based on one or more characteristic features.

**14.** A method, in particular as claimed in claim 12 or 13, for use with a treatment device (2) configured to perform a treatment operation on a body part of a subject, wherein the treatment device is configured to apply light pulses to skin of the body part to perform the treatment operation and to output a flash sound per light pulse, the method being configured to control a user interface to:

- display a first screen requesting user input regarding a treatment operation;
- obtain user input via the first screen, the user input comprising an image of a body part of a subject to be treated and/or size information of the size of the body part of the subject to be treated;
- display a second screen indicating connectivity of the apparatus or an electronic device comprising the apparatus with the treatment device;
- display a third screen indicating at least one of:

- an operation status of the skin treatment device,
- a flash sound counter counting the number of detected flash sounds since start of a treatment operation or a set or user-instructed moment,
- an indication if the flash sound counter exceeds a flash sound threshold, and

- an indication that the treatment operation has finished.

15. Computer program comprising program code means for causing a computer to carry out the steps of the method as claimed in claim 12, 13 or 14 when said computer program is carried out on the computer.

FIG.1

FIG.2

100

obtain audio signal ~ 101

segment audio signal ~ 102

extract characteristic feature(s) ~ 103

detect flash sounds ~ 104

increment flash sound counter ~ 105

output information ~ 106

FIG.3

200

```
obtain audio signal ⌇～201

        ↓

segment audio signal ⌇～202

        ↓

extract characteristic feature(s) ⌇～203

        ↓

derive flash sound information ⌇～204

        ↓

provide characteristic feature(s)
and flash sound information       ⌇～205
to algorithm / computing system
```

FIG.4

**FIG.5**

**FIG.6**

300

301 — Audio file → Extract chunks of audio — 303

302 — Annotated data → Basic Audio manipulations — 304

# FIG.7

400

401    402    403

audio → Pre - emphasis, frame blocking & windowing → Fast Fourier Transform (FFT) → Mel - Scale filter bank

MFCC ← Mel - Scale filter bank ← Log ←

406    405    404

# FIG.8

New audios and
annotations

↓

Fine tune models
on Flash dataset

↓

Continuous model update ⟲ Save best AI model

↓

Real time
treatment audio → AI model continuous
deployment on Mobile app → Count flashes

FIG.9

500

display first screen —501

obtain user input —502

display second screen —503

display third screen —504

FIG.10

FIG.11

Europäisches
Patentamt

European
Patent Office

Office européen
des brevets

# EUROPEAN SEARCH REPORT

**Application Number**

EP 22 15 6800

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/079972 A1 (SHENZHEN H&T INTELLIGENT CONTROL CO LTD [CN]) 2 May 2019 (2019-05-02) | 1-7,12, 15 | INV. A61B18/20 |
| Y | * paragraphs [0065] – [0108] * * figure 8 * | 11 | |
| X | JP H07 307812 A (SHARP KK) 21 November 1995 (1995-11-21) * paragraphs [0010], [0017] – [0021] * | 1,12,15 | |
| Y | EP 3 562 420 A1 (KONINKLIJKE PHILIPS NV [NL]) 6 November 2019 (2019-11-06) * paragraphs [0046], [0080], [0081] * | 11 | |
| A | US 2019/088367 A1 (STAMATOPOULOS CHARALAMPOS-CHRISTOS [GR] ET AL) 21 March 2019 (2019-03-21) * abstract * | 1,11,12, 15 | |

**TECHNICAL FIELDS SEARCHED (IPC)**

A61B

~~The present search report has been drawn up for all claims~~

| Place of search | Date of completion of the search | Examiner |
|---|---|---|
| Munich | 15 July 2022 | Kretschmann, Jana |

EPO FORM 1503 03.82 (P04C01)

Europäisches
Patentamt

European
Patent Office

Office européen
des brevets

**Application Number**

EP 22 15 6800

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7, 12(completely); 11, 15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
1. claims: 1-7, 12(completely); 11, 15(partially)

        an apparatus for use with a treatment device comprising an
        input for obtaining an audio signal, a processing unit
        configured to segment the audio signal, extract
        characteristic features, detect a flash sound and change a
        flash sound counter, and an output
                             ---


2. claims: 8, 9, 13(completely); 11, 15(partially)

        an apparatus for use with a treatment device comprising an
        input for obtaining an audio signal, a processing unit
        configured to segment the audio signal, extract
        characteristic features, derive annotation indicating flash
        sound and provide the information to an algorithm or
        computing system for training
                             ---


3. claims: 10, 14(completely); 11, 15(partially)

        an apparatus for use with a treatment device comprising an
        output for controlling a user interface to display a first
        screen, obtain user input, display a second screen and
        display a third screen
                             ---
```

## ANNEX TO THE EUROPEAN SEARCH REPORT
## ON EUROPEAN PATENT APPLICATION NO.

EP 22 15 6800

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2022

| Patent document cited in search report | | | Publication date | Patent family member(s) | | | Publication date |
|---|---|---|---|---|---|---|---|
| WO | 2019079972 | A1 | 02-05-2019 | CN | 109074822 | A | 21-12-2018 |
| | | | | WO | 2019079972 | A1 | 02-05-2019 |
| JP | H07307812 | A | 21-11-1995 | JP | 3251765 | B2 | 28-01-2002 |
| | | | | JP | H07307812 | A | 21-11-1995 |
| EP | 3562420 | A1 | 06-11-2019 | BR | 112019013133 | A2 | 24-12-2019 |
| | | | | BR | 112019013135 | A2 | 10-12-2019 |
| | | | | CN | 110114029 | A | 09-08-2019 |
| | | | | CN | 110121307 | A | 13-08-2019 |
| | | | | EP | 3562420 | A1 | 06-11-2019 |
| | | | | EP | 3562421 | A1 | 06-11-2019 |
| | | | | ES | 2906054 | T3 | 13-04-2022 |
| | | | | JP | 7080239 | B2 | 03-06-2022 |
| | | | | JP | 7080239 | B6 | 23-06-2022 |
| | | | | JP | 7126503 | B2 | 26-08-2022 |
| | | | | JP | 2020503118 | A | 30-01-2020 |
| | | | | JP | 2020503119 | A | 30-01-2020 |
| | | | | RU | 2741466 | C1 | 26-01-2021 |
| | | | | RU | 2019123598 | A | 29-01-2021 |
| | | | | US | 2019328455 | A1 | 31-10-2019 |
| | | | | US | 2021128939 | A1 | 06-05-2021 |
| | | | | WO | 2018122266 | A1 | 05-07-2018 |
| | | | | WO | 2018122270 | A1 | 05-07-2018 |
| | | | | WO | 2018122272 | A1 | 05-07-2018 |
| US | 2019088367 | A1 | 21-03-2019 | NONE | | | |

EPO FORM P0459

For more details about this annex : see Official Journal of the European Patent Office, No. 12/82